(19) Europäisches Patentamt
European Patent Office
Office européen des brevets

(11) Veröffentlichungsnummer : **0 497 735 A1**

(12) **EUROPÄISCHE PATENTANMELDUNG**

(21) Anmeldenummer : **92810038.7**

(22) Anmeldetag : **22.01.92**

(51) Int. Cl.$^5$ : **C07D 215/06,** C07D 215/18, C08K 5/3437, C10M 133/40

(30) Priorität : **31.01.91 CH 290/91**

(43) Veröffentlichungstag der Anmeldung : **05.08.92 Patentblatt 92/32**

(84) Benannte Vertragsstaaten : **BE DE FR GB IT NL**

(71) Anmelder : **CIBA-GEIGY AG**
**Klybeckstrasse 141**
**CH-4002 Basel (CH)**

(72) Erfinder : **Evans, Samuel, Dr.**
**Route des Charbonnières 17**
**CH-1723 Marly (CH)**

(54) **Hydrochinolinverbindungen.**

(57) Es werden neue, substituierte Tetrahydrochinolinverbindungen der Formel I

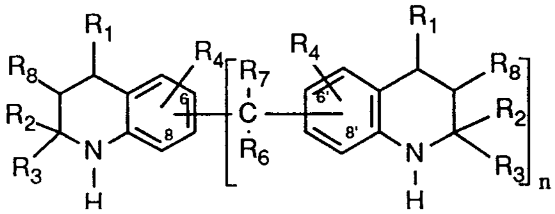

(I)

beschrieben, worin $R_1$ $C_{1-18}$Alkyl ist oder zusammen mit $R_8$ 1,4-Butylen bedeutet, $R_2$ und $R_3$ unabhängig voneinander $C_{1-18}$Alkyl, Phenyl oder Phenyl-$C_{1-4}$alkyl sind oder zusammen Tetra-, Penta- oder Hexamethylen bedeuten, $R_4$ Wasserstoff, Halogen, Nitro, $C_{1-24}$Alkyl oder eine Gruppe der Formel -O-$R_5$ bedeutet, $R_5$ Wasserstoff, $C_{1-18}$Alkyl oder Benzyl ist,
$R_6$ und $R_7$ unabhängig voneinander Wasserstoff, $C_{1-18}$Alkyl, durch -S- oder -O-unterbrochenes $C_{2-18}$Alkyl, Phenyl oder Phenyl-$C_{1-4}$alkyl bedeuten oder zusammen $C_{4-11}$Alkylen sind, $R_8$ Wasserstoff, $C_{1-18}$ Alkyl oder zusammen mit $R_1$ 1,4-Butylen ist und n 1, 2 oder 3 bedeutet. Die Verbindungen eignen sich als Stabilisatoren, insbesondere als Antioxidantien für organische Materialien.

EP 0 497 735 A1

Die Erfindung betrifft neue Hydrochinolinverbindungen, Zusammensetzungen, welche diese enthalten, sowie die Verwendung dieser Verbindungen als Stabilisatoren für organisches Material.

Aus der DE-A-2 243 777 sind Dihydrochinolinderivate der Formel

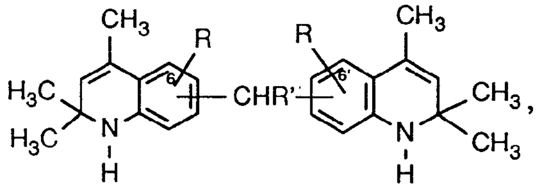

wobei R′ für Wasserstoff oder Alkyl mit 1 bis 4 C-Atomen und R für Methyl, das nicht an C6 oder C6′ gebunden ist, stehen, als Antioxidantien für Industrieprodukte, besonders Gummi- und Kautschukprodukte, bekannt. Später wurden Verbindungen dieser Grundstruktur auch als Konservierungsstoffe für Futtermittel (DE-A-3 540 195, WO-A-87/00 048, WO-A-88/08 420) sowie in Kombination mit organischen Halogeniden als Farbbildner in lichtempfindlichen Aufzeichnungsmaterialien (DD-A-155 212) vorgeschlagen. Ferner sind Tetrahydrochinolinverbindungen als Stabilisatoren für Schmiermittel aus den US-A-4,692,258 und 4,828,741 bekannt.

Überraschenderweise wurde nun gefunden, daß sich di- tri- und tetramere Tetrahydrochinolinderivate besonders gut als Stabilisatoren für organisches Material eignen.

Die Erfindung betrifft somit Verbindungen der Formel I

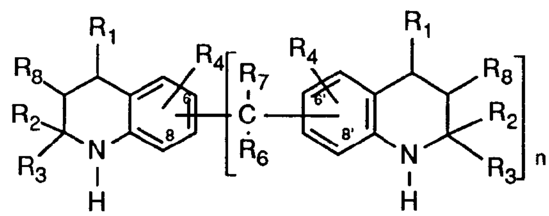 (I),

worin $R_1$ $C_{1-18}$Alkyl ist oder zusammen mit $R_8$ 1,4-Butylen bedeutet,

$R_2$ und $R_3$ unabhängig voneinander $C_{1-18}$Alkyl, Phenyl oder Phenyl-$C_{1-4}$alkyl sind oder zusammen Tetra-, Penta- oder Hexamethylen bedeuten,

$R_4$ Wasserstoff, Halogen, Nitro, $C_{1-24}$Alkyl oder eine Gruppe der Formel -O-$R_5$ bedeutet,

$R_5$ Wasserstoff, $C_{1-18}$Alkyl oder Benzyl ist,

$R_5$ und $R_7$ unabhängig voneinander Wasserstoff, $C_{1-18}$Alkyl, durch -S- oder -O- unterbrochenes $C_{2-18}$Alkyl, Phenyl oder Phenyl-$C_{1-4}$alkyl bedeuten oder zusammen $C_{4-11}$Alkylen sind,

$R_5$ Wasserstoff, $C_{1-18}$Alkyl oder zusammen mit $R_1$ 1,4-Butylen ist und n 1, 2 oder 3 bedeutet. Bevorzugt ist n 1 oder 2, insbesondere 1.

$R_1$, $R_2$, $R_3$, $R_4$, $R_6$, $R_7$, und $R_8$ in der Bedeutung von $C_{1-18}$Alkyl bzw. $C_{1-24}$Alkyl können gerad- oder verzweigtkettig sein und sind beispielsweise Methyl, Ethyl, Propyl, Isopropyl, n-Butyl, Isobutyl, t-Butyl, Pentyl, Isopentyl, Hexyl, Heptyl, 3- Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl bzw. zusätzlich noch beispielsweise Eicosyl oder Henicosyl. Bedeuten diese Reste Gruppen mit einer geringeren Zahl von C-Atomen, so sind entsprechende Beispiele ebenfalls obiger Liste zu entnehmen. Bedeuten die genannten Reste Alkyl, so haben sie bevorzugt 1-12, insbesondere 1-6, vor allem 1-4 C-Atome. Sind $R_6$ und $R_7$ von -O- oder -S- unterbrochen, so können diese eines oder mehrere der Heteroatome enthalten, und es finden sich darin beispielsweise die Gruppen $-CH_2-CH_2-O-$ und $-CH_2-CH_2-S-$, gegebenenfalls aneinandergereiht. Bevorzugt ist die Unterbrechung durch ein einzelnes O- oder S-Atom.

Bilden etwaige Reste zusammen Alkylen, so kann das beispielsweise ein lineares oder verzweigtes, vorzugsweise lineares Alkylen bedeuten. Beispiele für solche Reste ergeben sich aus den obigen Beispielen für $C_{1-18}$Alkyl mit der entsprechenden Anzahl der C-Atome durch Anfügen des Suffixes -en. Bevorzugt sind $R_5$ und $R_7$ zusammen Tetra-, Penta- und Hexamethylen, insbesondere Pentamethylen.

$R_2$, $R_3$, $R_5$ und $R_7$ als Phenyl-$C_1$-$C_4$-alkyl bedeuten z.B. Benzyl, Phenethyl, 3-Phenylpropyl, $\alpha$-Methylbenzyl oder $\alpha$,$\alpha$-Dimethylbenzyl. Bevorzugt ist Benzyl.

$R_4$ und $R_8$ sind vorzugsweise Wasserstoff.

Bevorzugte Verbindungen der Formel I sind solche, worin worin $R_1$ $C_{1-8}$Alkyl, $R_2$ und $R_3$ unabhängig voneinander $C_{1-8}$Alkyl, Phenyl, Benzyl oder Cyclohexyl sind, $R_4$ Wasserstoff, $C_{1-12}$Alkyl, Hydroxy, Methoxy oder Ethoxy ist, $R_6$ und $R_7$ Wasserstoff, $C_{1-12}$Alkyl, Phenyl oder zusammen Pentamethylen bedeuten, und $R_8$ Wasserstoff ist.

Bevorzugt sind auch diejenigen Verbindungen der Formel I, worin $R_1$ , $R_2$ und $R_3$ Methyl sind, $R_4$ Wasserstoff oder $C_{1-12}$Alkyl ist, $R_6$ Wasserstoff oder $C_{1-12}$Alkyl bedeutet, $R_7$ für Wasserstoff, Methyl, Ethyl oder Phenyl steht, $R_8$ Wasserstoff ist, und n 1 ist, wobei $R_4$, $R_6$, $R_7$ und $R_8$ insbesondere Wasserstoff bedeuten.

$R_4$ steht in Formel I bevorzugt an den Positionen 6(6') oder 8(8'), und die Gruppe

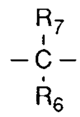

verbindet im Fall n = 1 vorzugsweise die Positionen 8 und 8' oder 6 und 6'. Besonders bevorzugt steht $R_4$ an Position 6 (6').

Ist $R_4$ Wasserstoff und n 1, dann verbindet die Gruppe

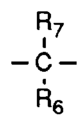

bevorzugt die C-Atome 6 und 6'.

Im Fall n = 2 nehmen die Reste

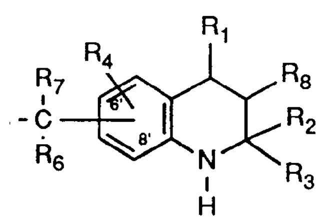

bevorzugt die 6- und 8-Positionen ein.

In besonders bevorzugten Verbindungen der Formel I sind $R_1$, $R_2$ und $R_3$ Methyl, $R_4$, $R_6$ und $R_6$ Wasserstoff, $R_7$ steht für Wasserstoff, Phenyl oder $C_6$-$C_{18}$-Alkyl, n für 1 und die Gruppe

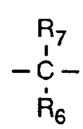

ist mit den C-Atomen 6 und 6' verbunden.

Die Verbindungen der Formel I eignen sich hervorragend zum Stabilisieren von organischen Materialien gegen lichtinduzierten, thermischen oder /und oxidativen Abbau. Gegenstand der Erfindung sind daher auch Zusammensetzungen enthaltend ein gegen solche Abbaureaktionen empfindliches organisches Material und mindestens eine Verbindung der Formel I bzw. die Verwendung von Verbindungen der Formel I als Stabilisatoren für organische Materialien gegen die genannten Abbauarten.

Die Verbindungen der Formel I können insbesondere als Stabilisatoren für natürliche, halbsynthetische oder synthetische Polymere, besonders thermoplastische Kunststoffe und Elastomere, sowie für funktionelle Müssigkeiten, insbesondere Schmierstoffe und Hydraulikflüssigkeiten, eingesetzt werden. Beispiele für solche Substrate sind der folgenden Aufzählung von geeigneten Materialien zu entnehmen.

1. Polymere von Mono- und Diolefinen, beispielsweise Polypropylen, Polyisobutylen, Polybuten- 1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Statistische oder alternierende Copolymere von $\alpha$-Olefinen mit Kohlenmonoxid.
3b. Kohlenwasserstoffharze (z.B. $C_5$-$C_9$) inklusive hydrierte Modifikationen davon (z.B. Klebrigmacherharze).
4. Polystyrol, Poly-(p-methylstyrol), Poly-($\alpha$-methylstyrol).
5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von $\alpha,\beta$-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

28. Naturliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wäßrige Emulsionen.

29. Wäßrige Emulsionen natürlicher oder synthetischer Kautschuke, wie z.B. Naturkautschuk-Latex oder Latices von carboxylierten Styrol-Butadien-Copolymeren.

In den erfindungsgemäßn Zusammensetzungen sind die Verbindungen der Formel I zweckmäßig zu 0.01 bis 10, beispielsweise zu 0.05 bis 5, vorzugsweise zu 0.05 bis 3, insbesondere jedoch zu 0.1 bis 2 Gew.-% enthalten. Es kann sich dabei um eine oder mehrere dieser Verbindungen der Formel I handeln, und die Gewichtsprozentangaben beziehen sich auf die gesamte Menge dieser Verbindungen. Berechnungsgrundlage ist dabei das Gesamtgewicht des organischen Materials ohne die Verbindungen der Formel I.

Die Einarbeitung in die Materialien kann beispielsweise durch Einmischen oder Aufbringen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor, während oder unmittelbar nach der Polymerisation der entsprechenden Monomeren bzw. vor der Vernetzung. Die Verbindungen der Formel I können dabei als solche, aber auch in encapsulierter Form (z.B in Wachsen, Ölen oder Polymeren) zugesetzt werden. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2.5 bis 25 Gew.-% enthält den zu stabilisierenden Kunststoffen zugesetzt werden.

Zweckmäßig kann die Einarbeitung der Verbindungen der Formel I nach folgenden Methoden erfolgen:

– als Emulsion oder Dispersion (z.B. zu Latices oder Emulsionspolymeren)
– Als Trokenmischung während des Vermischens von Zusatzkomponenten oder Polymermischungen
– durch direktes Zugeben in die Verarbeitungsapparatur (z.B. Extruder, Innenmischer usw.)
– als Lösung oder Schmelze.

Erfindungsgemäß Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Die Erfindung betrifft auch ein Verfahren zum Stabilisieren von organischem Material, insbesondere von thermoplastischen Polymeren, Elastomeren oder funktionellen Flüssigkeiten, vor allem Schmierstoffen, gegen oxidativen, thermischen und/oder lichtinduzierten Abbau, dadurch gekennzeichnet, daß man diesem Material als Stabilisatoren Verbindungen der Formel I zusetzt bzw. auf dieses aufbringt.

Die Verbindungen der Formel I sind beispielsweise besonders geeignet, um funktionellen Flüssigkeiten verbesserte Gebrauchseigenschaften zu verleihen. Es ist dabei insbesondere auf ihre überraschend gute Wirkung als Antioxidantien hinzuweisen. Deshalb umfaßt die Erfindung auch Zusammensetzungen, enthaltend eine funktionelle Flüssigkeit und wenigstens eine Verbindung der allgemeinen Formel 1, wie oben beschrieben.

Als funktionelle Flüssigkeiten sind beispielsweise Schmierstoffe, Hydraulikflüssigkeiten und Metallbearbeitungsflüssigkeiten zu nennen.

Die in Frage kommenden Schmierstoffe basieren beispielsweise auf mineralischen oder synthetischen Ölen oder Mischungen davon oder auf pflanzlichen und tierischen Ölen, Fetten und Wachsen. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (D. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Als pflanzliche Schmierstoffe sind die Öle, Fette und Wachse, gewonnen beispielsweise aus Oliven, Palmen, Palmkernen, Rüben, Raps, Leinen, Nüssen, Soja, Baumwolle, Ricinus, Sonnenblumen, Kürbiskernen, Kokos, Mais oder deren modifizierte Formen, z.B geschwefelte oder epoxidierte Öle, wie epoxidiertes Sojaöl, sowie Mischungen der Substanzen zu nennen. Beispiele für tierische Öle, Fette und Wachse, die als Schmierstoffe angewendet werden können, sind Talge, Fischöle, Spermöl, Klauenöl, Trane und Specköle, deren modifizierte Formen und Mischungen.

Metallbearbeitungsflüssigkeiten wie Walz-, Zieh- und Schneidöle basieren zumeist auf den oben beschriebenen Mineral- und synthesichen Ölen und können auch als Öl-in-Wasser- bzw. Wasser-in-Öl-Emulsionen vorliegen.Das gleiche gilt für Hydraulikflüssigkeiten. Als weitere funktionelle Flüssigkeiten kommen z.B. Kompressoröle und Spinnpräparationen in Frage.

Die Verbindungen der Formel I, wie oben beschrieben, können z.B. in Mengen von 0,01 bis 10 Gew.-%, zweckmäßig in Mengen von 0.03 bis 5 Gew.-%, vorzugsweise in einer Menge von 0.05 bis 3 Gew.-% und ganz besonders bevorzugt von 0.5 bis 1.5 Gew.-%, bezogen auf die Zusammensetzung, in der funktionellen Flüssigkeit vorliegen.

Die Verbindungen der Formel I können der funktionellen Flüssigkeit auf an sich bekannte Weise beigemischt werden. Die Verbindungen sind beispielsweise in Ölen gut löslich. Es ist auch möglich, einen sogenannten Masterbatch herzustellen, der nach Maßgabe des Verbrauchs auf Einsatzkonzentrationen mit der

entsprechenden funktionellen Flüssigkeit verdünnt werden kann.

Zusätzlich zu den erfindungsgemäßen Verbindungen bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:

1. Antioxidantien

1. 1. Alkylierte Monophenole, z.B. 2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol 2,6-Di-tert-butyl-4-methoxymethylphenol, o-tert-Butylphenol.

1.2. Alkylierte Hydrochinone, z.B. 2,6-Di-tert.butyl-4-methoxyphenol, 2,5-Di-tert.butylhydrochinon, 2,5-Di-tert.amyl-hydrochinon, 2,6-Diphenyl-4-octadecyloxyphenol.

1.3. Hydroxylierte Thiodiphenylether, z.B. 2,2′-Thio-bis-(6-tert.-butyl-4-methylphenol), 2,2′-Thio-bis-(4-octylphenol), 4,4′-Thio-bis-(6-tert.butyl-3-methylphenol), 4,4′-Thio-bis-(6-tert.butyl-2-methylphenol).

1.4. Alkyliden-Bisphenole, z.B. 2,2′-Methylen-bis-(6-tert-butyl-4-methylphenol), 2,2′-Methylen-bis-(6-tert-butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert-butylphenol), 2,2′-Ethyliden-bis-(4,6-di-tert-butylphenol), 2,2′-Ethyliden-bis-(6-tert-butyl-4- oder -5-iso-butylphenol), 2,2′-Methylen-bis-[6-(α-methylbenzyl)-4-nonylphenol], 2,2′-Methylen-bis-[6(α,α-dimethylbenzyl)-4-nonylphenol], 4,4′-Methylen-bis-(2,6-di-tert-butylphenol), 4,4′-Methylen-bis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3′-tert-butyl-4′-hydroxyphenyl)-butyrat], Bis-(3-tert-butyl-4-hydroxy-5-methylphenyl)-dicyclopentadien, Bis-[2-(3′-tert-butyl-2′-hydroxy-5′-methyl-benzyl)-6-tert-butyl-4-methyl-phenyl]-terephthalat.

1.5 Benzylverbindungen, z.B. 1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 3,5-Di-tert-butyl-4-hydroxybenzyl-phosphonsäure-dioctadecylester, 3,5-Di-tert-butyl-4-hydroxybenzyl-phosphonsäure-monoethylester, Calcium-salz.

1.6. Acylaminophenole, z.B. 4-Hydroxy-laurinsäureanilid, 4-Hydroxy-sterinsäureanilid, 2,4-Bis-octylmercapto-6-(3,5-di-tert-butyl-4-hydroxyanilino)-s-triazin, N-(3,5-di-tert-butyl-4-hydroxyphenyl)-carbaminsäureoctylester.

1.7. Ester der β-(3,5-Di-tert-butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N′-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

1.10. Amide der β-(3,5-Di-tert.butyl-4-hydroxyphenyl)-propionsäure, wie z.B. N,N′-Bis-(3,5-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert-butyl-4-hydroxyphenylpropionyl)-hydrazin.

2. UV-Absorber und Lichtschutzmittel

2.1. 2-(2′-Hydroxyphenyl)-benztriazole, wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl, 4′-Octoxy-, 3′,5′-Di-tert.amyl-, 3′,5′-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

2. 3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2′-Thio-bis-[4-(1,1,3,3-tetramethylbutyl)-phenols],

wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl-oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzylmalonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1′-(1,2-Ethandiyl)-bis-(3,3,5,5-tetramethyl-piperazinon).

2.7. Oxalsäurediamide, wie z.B. 4,4′-Di-octyloxy-oxanilid, 2,2′-Di-octyloxy-5,5′-di-tert.-butyl-oxanilid, 2,2′-Di-dodecyloxy-5,5′di-tert.butyl-oxanilid, 2-Ethoxy-2′-ethyl-oxanilid, N,N′-Bis-(3-dimethylaminopropyl)-oxalamid, 2-Ethoxy-5-tert-butyl-2′ethyloxanilid und dessen Gemisch mit 2-Ethoxy-2′-ethyl-5,4′-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-methylphenyl)-1,3,5-triazin.

3. Metalldesaktivatoren, wie z.B. N,N′-Diphenyloxalsäurediamid, N-Salicylal-N′-salicyloylhydrazin, N,N′-Bis-(salicyloyl)-hydrazin, N,N′-Bis-(3,5-di-tert-butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Tristearyl-sorbit-triphosphit, Tetrakis-(2,4-di-tert-butylphenyl)-4,4′-biphenylen-diphosphonit, 3,9-Bis-(2,4-di-tert-butylphenoxy)-2,4,8,10-tetraoxa-3,9-diphosphaspiro[5.5]undecan.

5.Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-butyl-dithiocarbamat, Dioctadecyldisulfid, Pentaerythrit-tetrakis-(β-dodecylmercapto)-propionat.

6.Polyamidstabilisatoren, wie z.B. Kupfersalze in Kombination mit Jodiden und/oder Phosphorverbindungen und Salze des zweiwertigen Mangans.

7.Basische Co-Stabilisatoren, wie z.B. Melamin, Polyvinylpyrrolidon, Dicyandiamid, Triallylcyanurat Harnstoff-Derivate, Hydrazin-Derivate, Amine, Polyamide, Polyurethane, Alkali - und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8.Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemäßen Zusammensetzungen um solche auf Basis von funktionellen Flüssigkeiten, insbesondere Schmierstoffen und Hydraulikflüssigkeiten bzw. Metallbearbeitungsflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleißschutzadditive. Beispiele hierfür sind:

Beispiele für phenolische Antioxidantien:

Solche können den vorstehenden Punkten 1. 1. bis 1. 10 entnommen werden.

Beispiele für aminische Antioxidantien:

N,N′-Di-isopropyl-p-phenylendiamin, N,N′-Di-sec-butyl-p-phenylendiamin, N,N′-Bis(1,4-dimethyl-pentyl)-p-phenylendiamin, N,N′-Bis-(1-ethyl-3-methyl-pentyl)-p-phenylendiamin, N,N′-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, 2,3-Dihydro- 3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbemsteinsäure-anhydrid, Alkenylbernsteinsäure-Teilester und -Teilamide, 4-Nonylphenoxy-essigsäure.
b) Stickstoffhaltige Verbindungen, z.B.:
i. Primäre, sekundäre oder tertiäre aliphatische oder cycloaliphatische Amine und Amin-Salze von organischen und anorganischen Säuren, z.B. öllösliche Alkylammoniumcarboxylate.
ii. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleißschutz-Additive sind:

Schwefel und/oder Phosphor und/oder Halogen enthaltende Verbindungen, wie geschwefelte pflanzliche Öle, Zinkdialkyldithiophosphate, Tritolylphosphat chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Die vorliegende Erfindung betrifft auch die Verwendung von Verbindungen der Formel I zum Stabilisieren von gegen oxidativen, thermischen und/oder lichtinduzierten Abbau empfindlichem organischem Material, insbesondere von natürlichen oder (halb)synthetischen Polymeren oder von funktionellen Flüssigkeiten, vor allem von thermoplastischen Polymeren oder Elastomeren und von Schmierstoffen. Die Verbindungen wirken beispielsweise besonders gut als Antioxidantien in funktionellen Flüssigkeiten, wie oben erwähnt.

Zweckmäßige und bevorzugte Verbindungen der Formel I, wie vorstehend beschrieben führen zu zweckmäßigen und bevorzugten Zusammensetzungen.

Die Verbindungen der Formel I werden nach an sich bekannten Methoden hergestellt (vgl. z.B. WO-A-88/08 420, DE-A-2 243 777):

Die Synthese folgt beispielsweise dem Schema

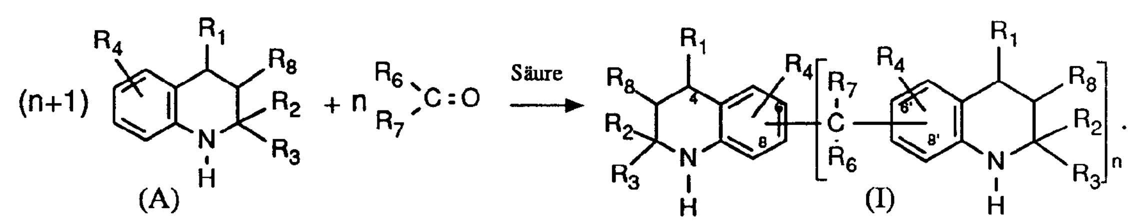


(A)

(I)

Wie in DE-A-2 243 777 ausgeführt, können bei der Umsetzung Gemische entstehen, in denen Verbindungen der Formel I mit n = 1, 2, und 3 nebeneinander vorliegen. Die Ausbeute an Hauptprodukten läßt sich anhand von Reaktionsbedingungen und Mengenverhältnissen der Ausgangsstoffe weitgehend steuern. Die entstandenen Gemische können nach bekannten Verfahren (wie Kristallisation, fraktionierte Destillation oder Chromatographie) getrennt werden oder auch vorteilhaft als solche ihrem Zweck zugeführt werden.

In einer besonders bevorzugten Ausführungsform geht man z.B. von 2,2,4-Trimethyl-1,2,3,-tetrahydrochinolin aus und setzt dieses mit einer Carbonylverbindung $R_6COR_7$ in Gegenwart eines 1-2-fachen Überschusses einer Mineralsäure und 2-3 Gew.% einer stickstoffhaltigen Base zu der Verbindung der Formel I um, worin n 1 ist, $R_4$ und $R_6$ Wasserstoff bedeuten und $R_1$, $R_2$ und $R_3$ Methyl sind.

Die Ausgangsverbindungen der Formel A sind bekannt oder können nach an sich bekannten Verfahrn erhalten werden, wie z.B. in US-A-4,692,258 und US-A-4,828,741 beschrieben.

Die folgenden Beispiele erläutern die Erfindung weiter, ohne sie jedoch zu beschränken. Alle Teile- und Prozentangaben beziehen sich darin - ebenso wie in der übrigen Beschreibung - auf das Gewicht, sofern nicht anders angegeben.

Beispiel 1:

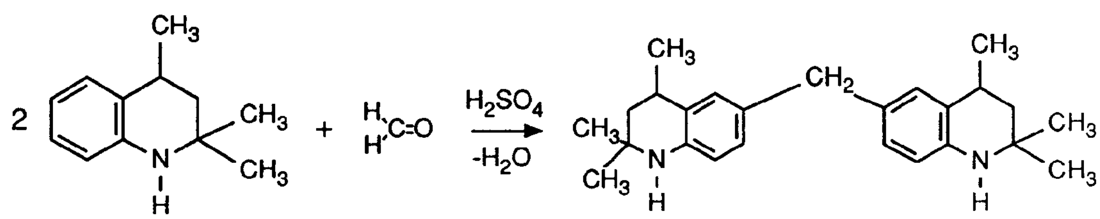


35.2 g (0.2 mol) 2,2,4-Trimethyl-1,2,3,4-tetrahydrochinolin werden in 100 ml Wasser bei Raumtemperatur in einem 300 ml Sulkierkolben vorgelegt, wobei sich zwei Phasen bilden. Nachdem innerhalb von 15 Minuten 10.2 g (0.1 mol) konzentrierte Schwefelsäure zugetropft sind, entsteht unter leichter Wärmeentwicklung eine klare, bräunliche Lösung. Nach Kühlung im Eisbad werden 8.4 g (0.1 mol) 36 %ige Formaldehydlösung innerhalb von 30 Minuten zugetropft. Nach einstündigem Rühren läßt man auf Raumtemperatur erwärmen. Nach weiteren 16 Stunden Rühren entsteht ein brauner Kristallbrei. Dieser wird in 200 ml Toluol aufgenommen und mit verdünnter Kalilauge etwas über den Neutralpunkt versetzt. Die organische Phase wird mit 300 ml Wasser neutral gewaschen. Nach Trocknen über 10 g $Na_2SO_4$ wird filtriert und das Filtrat am Rotationsverdampfer zur Trockne eingeengt. Nach 2-stündigem Trocknen im Hochvakuum erhält man 35.8 g (98.8% d.Th.) eines braunen, harzigen Öls.

| Analyse: | | gef. | ber. |
|---|---|---|---|
| | C: | 82.7% | 82.2% |
| | H: | 9.5% | 9.5% |
| | N | 7.7% | 7.7% |

NMR Messungen bestätigen die Stellung der Methylenbrücke.

Beispiel 2:

Test auf Stabilisierung eines industriellen Öls gegen oxidativen Abbau

(TFOUT: Thin Film Oxygen Uptake Test)

Dieser Test ist eine modifizierte Form des Rotary Bomb Test für Mineralöle (ASTM D 272). Eine detaillierte Beschreibung findet sich bei C.S.Ku, S.M.Hsu, Lubrication Engineering 40 (1984) 75-83. Das Testöl ist in diesem Fall ein kommerzielles Motorenöl vom Typ ARAL RL 136/3 Das zu testende Additiv (Verbindung der Formel I) wird im Öl in Gegenwart von Wasser (2 %), einer oxydierten/nitrierten Benzinfraktion (4 %) und eines Gemisches von flüssigen Metallnaphthenaten (4 %) bei 6.1 bar Sauerstoffdruck und 160°C auf seine stabilisierende Wirkung getestet. Das Wasser und die beiden flüssigen Katalysatoren für den Test werden unter der Bezeichnung Standard Reference Material 1817 vom National Bureau of Standards (NBS) bezogen, mit Bescheinigung für die Analyse. Der Test ist abgeschlossen, wenn im Druck/Zeit-Diagramm ein deutlicher Knick die einsetzende Oxidation am Ende der Induktionsperiode (min.) anzeigt. Eine lange Induktionsperiode bedeutet eine gute stabilisierende Wirkung des Additivs. Die Resultate sind in der Tabelle 1 zusammengefaßt.

Tabelle 1

| Verbindung aus Beispiel | Zusatzmenge (Gew. %) | Induktionsperiode (min.) |
|---|---|---|
| 1 | 0.75 | 289 |
| Referenz | ohne Additiv | 146 |

**Patentansprüche**

1. Verbindungen der Formel I

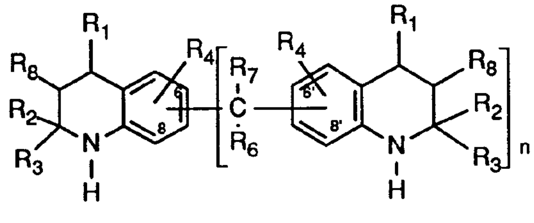

(I),

worin $R_1$ $C_{1-18}$Alkyl ist oder zusammen mir $R_8$ 1,4-Butylen bedeutet,
$R_2$ und $R_3$ unabhängig voneinander $C_{1-18}$Alkyl, Phenyl oder Phenyl-$C_{1-4}$alkyl sind oder zusammen Tetra-, Penta- oder Hexamethylen bedeuten,
$R_4$ Wasserstoff, Halogen, Nitro, $C_{1-24}$Alkyl oder eine Gruppe der Formel -O-$R_5$ bedeutet,

$R_5$ Wasserstoff, $C_{1-18}$Alkyl oder Benzyl ist,
$R_6$ und $R_7$ unabhängig voneinander Wasserstoff, $C_{1-18}$Alkyl, durch -S- oder -O-unterbrochenes $C_{2-18}$Alkyl, Phenyl oder Phenyl-$C_{1-4}$alkyl bedeuten oder zusammen $C_{4-11}$Alkylen sind,
$R_8$ Wasserstoff, $C_{1-18}$Alkyl oder zusammen mir $R_1$ 1,4-Butylen ist und n 1, 2 oder 3 bedeutet.

**2.** Verbindungen nach Anspruch 1, worin n 1 ist.

**3.** Verbindungen nach Anspruch 1, worin $R_1$ $C_{1-8}$Alkyl, $R_2$ und $R_3$ unabhängig voneinander $C_{1-8}$Alkyl, Phenyl, Benzyl oder Cyclohexyl sind, $R_4$ Wasserstoff, $C_{1-12}$Alkyl, Hydroxy, Methoxy oder Ethoxy ist, $R_6$ und $R_7$ Wasserstoff, $C_{1-12}$Alkyl, Phenyl oder zusammen Pentamethylen bedeuten, und $R_8$ Wasserstoff ist.

**4.** Verbindungen nach Anspruch 2, worin $R_1$ , $R_2$ und $R_3$ Methyl sind, $R_4$ Wasserstoff oder $C_{1-12}$Alkyl ist, $R_8$ Wasserstoff oder $C_{1-12}$Alkyl bedeutet, $R_7$ für Wasserstoff, Methyl, Ethyl oder Phenyl steht, $R_8$ Wasserstoff ist, und n 1 ist.

**5.** Verbindungen nach Anspruch 2, worin $R_1$, $R_2$ und $R_3$ Methyl sind, $R_4$, $R_6$ und $R_8$ Wasserstoff sind $R_7$ für Wasserstoff, Phenyl oder $C_6$-$C_{18}$-Alkyl steht, und die Gruppe

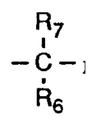

mit den C-Atomen 6 und 6′ verbunden ist.

**6.** Verbindungen nach Anspruch 4, worin $R_4$ ,$R_8$, und $R_7$ Wasserstoff sind.

**7.** Verbindungen nach Anspruch 1, worin $R_4$ in Formel I an den Positionen 6 (6′) oder 8 (8′) sitzt, n 1 oder 2 ist, und die Gruppe

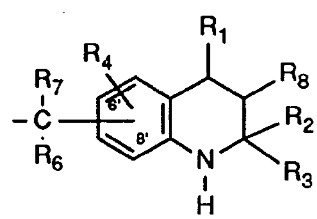

mit den C-Atomen 8 und/oder 6 verbunden ist.

**8.** Verbindungen nach Anspruch 1, worin $R_4$ in Formel I die Position 6 (6′) einnimmt.

**9.** Verbindungen nach Anspruch 1, worin n 1 und $R_4$ Wasserstoff ist, und die Gruppe

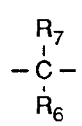

mit den C-Atomen 6 und 6′ verbunden ist.

**10.** Verbindungen nach Anspruch 1, worin $R_4$ an den Positionen 6 und 6′ zu finden ist, die Gruppe

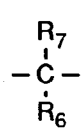

mit den C-Atomen 8 und 8′ verbunden ist, und n 1 ist.

11. Zusammensetzungen enthaltend
A) ein gegen lichtinduzierten, thermischen oder /und oxidariven Abbau empfindliches organisches Material und
B) mindestens eine Verbindung der Formel I gemäß Anspruch 1.

12. Zusammensetzungen nach Anspruch 11, worin das organische Material ein natürliches, halbsynthetisches oder synthetisches Polymer oder eine funktionelle Flüssigkeit ist.

13. Zusammensetzungen nach Anspruch 11, worin das organische Material ein synthetisches, insbesondere ein thermoplastisches, Polymer oder ein Elastomer ist.

14. Zusammensetzungen nach Anspruch 11, worin das organische Material ein Schmierstoff oder eine Hydraulikflüssigkeit ist.

15. Verwendung von Verbindungen der Formel I gemäß Anspruch 1 zum Stabilisieren von gegen oxidativen, thermischen und/oder lichtinduzierten Abbau empfindlichem organischen Material.

16. Verwendung nach Anspruch 15 zum Stabilisieren von natürlichen, halbsynthetischen oder synthetischen Polymeren oder von funktionellen Flüssigkeiten.

17. Verwendung nach Anspruch 15 zum Stabilisieren von thermoplastischen Polymeren oder Elastomeren.

18. Verwendung nach Anspruch 15 zum Stabilisieren von Schmierstoffen oder Hydraulikflüssigkeiten.

19. Verfahren zum Stabilisieren von organischem Material gegen oxidativen, thermischen und/oder lichtinduzierten Abbau, dadurch gekennzeichnet, daß man diesem Material als Stabilisaroren Verbindungen der Formel I zusetzt bzw. auf dieses aufbringt.

20. Verfahren nach Anspruch 19 zum Stabilisieren von thermoplastischen Polymeren, Elastomeren oder funktionellen Flüssigkeiten, insbesondere von Schmierstoffen.

Europäisches Patentamt

# EUROPÄISCHER RECHERCHENBERICHT

Nummer der Anmeldung

EP 92 81 0038

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A<br>D | EP-A-0 273 868 (CIBA-GEIGY AG)<br>* Seite 2 - Seite 6, Zeile 25 *<br>& US-A-4 828 741 | 1,18 | C07D215/06<br>C07D215/18<br>C08K5/3437<br>C10M133/40 |
| A,D | DE-A-2 243 777 (MATERIAL VEGYI KSZ,BUDAPEST)<br>* Seite 6, Absatz 2 - Seite 9, Absatz 4 * | 1,15 | |
| A | DE-A-2 832 126 (BRIDGESTONE TIRE CO.LTD.)<br>* Seite 1 - Seite 2; Ansprüche 1,2,3 * | 1,15 | |
| A | FR-A-2 355 046 (THE B.F.GOODRICH COMPANY)<br>* Seite 1 - Seite 5, Zeile 16 * | 1,15 | |
| A | US-A-4 244 864 (ROBERT H.CAMPBELL ET AL.)<br>* Spalte 1 - Spalte 3, Zeile 32 * | 1,15 | |

RECHERCHIERTE SACHGEBIETE (Int. Cl.5 )

C07D
C08K
C10M

Der vorliegende Recherchenbericht wurde für alle Patentansprüche erstellt

| Recherchenort | Abschlußdatum der Recherche | Prüfer |
|---|---|---|
| BERLIN | 13 MAI 1992 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)